# FASCICULE DE BREVET EUROPEEN

(11) **EP 2 379 752 B1**
(45) Date de publication et mention de la délivrance du brevet: **30.09.2015**
(21) Numéro de dépôt: 10707312.4
(22) Date de dépôt: 18.01.2010
(51) Int. Cl.: C12Q 1/68

(54) **PROCEDES POUR DETERMINER LA SUSCEPTIBILITE A CONTRACTER UNE INFECTION NOSOCOMIALE CHEZ UN PATIENT ET POUR ETABLIR UN PRONOSTIC D'EVOLUTION D'UN SYNDROME SEPTIQUE**
VERFAHREN ZUR BESTIMMUNG DER ANFÄLLIGKEIT EINES SUBJEKTS FÜR EINE NOSOKOMIALE INFEKTION UND ZUR PROGNOSE DER ENTWICKLUNG EINES SEPTISCHEN SYNDROMS
METHODS FOR DETERMINING THE SUSCEPTIBILITY OF A SUBJECT TO CONTRACT A NOSOCOMIAL INFECTION AND FOR ESTABLISHING A PROGNOSE OF EVOLUTION OF SEPTIC SYNDROME

(30) Priorité: 19.01.2009 FR 0950306; 19.01.2009 FR 0950305
(43) Date de publication de la demande: 26.10.2011
(73) Titulaire: Biomérieux S.A., 69280 Marcy L'Etoile (FR); Hospices Civils de Lyon, 69002 Lyon (FR)
(72) Inventeur: CAZALIS, Marie-Angélique, F-69120 Vaulx en Velin (FR); LEPAPE, Alain, F-69230 Saint-Genis Laval (FR); MONNERET, Guillaume, F-69003 Lyon (FR); MOUGIN, Bruno, F-69003 Lyon (FR); PACHOT, Alexandre, F-01400 Sulignat (FR)
(86) Numéro de dépôt international: PCT/FR2010/050070
(87) Numéro de publication internationale: WO 2010/082004

(56) Documents cités:
- EP-A1- 1 318 403
- EP-A1- 1 950 310
- WO-A2-2006/015260
- US-A1- 2007 092 911
- US-A1- 2008 070 235
- GREENBAUM D ET AL: "COMPARING PROTEIN ABUNDANCE AND MRNA EXPRESSION LEVELS ON A GENOMIC SCALE", GENOME BIOLOGY (ONLINE), BIOMED CENTRAL LTD, GB, vol. 40, no. 9, 1 January 2003 (2003-01-01), pages 117.01-117.08, XP008036618, ISSN: 1465-6914

## Description

La présente invention concerne un procédé pour déterminer la susceptibilité à contracter une infection nosocomiale chez un patient. L'invention a aussi pour objet un procédé pour l'établissement d'un pronostic d'évolution d'un choc septique chez un patient.

Les infections nosocomiales constituent un réel problème de santé publique. En France, on estime entre 500 000 et 800 000 le nombre de patients qui contractent chaque année une infection nosocomiale. L'apparition des infections nosocomiales est liée à différents facteurs, tels que les techniques médicales utilisées, mais aussi à la susceptibilité du patient à contracter une telle infection nosocomiale. Ainsi, les patients ayant un système immunitaire déficient, les personnes dénutries, les nouveaux-nés, les personnes âgées, les personnes en syndrome septique, les grands brûlés, les personnes en état traumatique peuvent être plus prédisposées que d'autres à contracter une infection nosocomiale.

Le syndrome septique, réponse systémique à l'infection, représente l'une des premières causes de mortalité dans les services de réanimation. Il peut résulter d'une infection bactérienne, virale, mycosique ou parasitaire. Ces syndromes septiques peuvent être classés en fonction de leur gravité. On distingue par ordre croissant de gravité le SIRS (Systemic Inflammatory Response Syndrome), le sepsis, le sepsis sévère et le choc septique. Un groupe d'experts a ainsi proposé en 2001 des critères de définitions de ces quatre syndromes cliniques^{[1]} :
- le SIRS, est une réponse systémique inflammatoire déclenchée par une variété de causes infectieuses ou non. Parmi les états de SIRS déclenchés par des causes non infectieuses, on peut citer les états traumatiques, les brûlures, les pancréatites, les syndromes respiratoires aiguës. Une réponse systémique inflammatoire se manifestant par au moins deux des signes suivants : a) température supérieure à 38°C ou inférieure à 36°C ; b) rythme cardiaque supérieur à 90 battements par minute ; c) rythme respiratoire supérieur à 20 respirations par minute ; d) nombre de leucocytes supérieur à 12000/mm³ ou inférieur à 4000/mm³,
- le sepsis est un syndrome de réponse systémique inflammatoire en relation avec une infection,
- un sepsis sévère est un sepsis associé à une hypotension artérielle et/ou hypoperfusion et/ou dysfonctionnement d'au moins un organe,
- le choc septique est un sepsis sévère associé à une hypotension persistante et peut être qualifié par :
   - la présence d'un site infectieux identifié,
   - une hypotension persistante malgré des remplissages adéquats et des traitements vasopresseurs.

D'une manière générale, les signes d'un sepsis, d'un sepsis sévère et d'un choc septique sont proches, et la différence entre ces trois situations réside principalement dans l'importance de la perturbation de toutes les fonctions vitales. Lors d'un choc septique, on observe principalement une chute de pression artérielle, tachycardie, polypnée, marbrures cutanées, hypo- ou hyperthermie, frissons. Ces signes s'accompagnent également d'un dysfonctionnement d'organes "cibles" avec une altération de fonction d'organes à distance du foyer infectieux (reins, poumons, système nerveux central, appareil digestif et les systèmes de la coagulation du sang) se traduisant par une oligurie (<0,5 ml/kg/h), insuffisance rénale, hypoxémie, thrombopénie, agitation, confusion).

Les patients en syndrome septique sont également plus ou moins sensibles aux infections nosocomiales, c'est à dire les infections en relation avec un séjour dans un établissement de santé. Cette sensibilité a un impact considérable sur la survie et le bon rétablissement de ces patients.

L'évolution d'un syndrome septique depuis le stade de sepsis vers un stade de sepsis sévère, puis de choc septique n'est pas systématique puisque environ 64% des patients septiques développent un sepsis sévère, et 23% des patients en sepsis sévère évoluent en choc septique. Avant cette étape ultime de choc septique, des traitements doivent être prescrits au patient afin d'interrompre et d'inverser le processus physiopathologique. Il faut ainsi restaurer un état hémodynamique satisfaisant et assurer une ventilation efficace. Il faut également mener de front le traitement symptomatique du choc et un traitement antibiotique adapté dès que possible en fonction des données bactériologiques.

Il apparaît que si certains patients développant un syndrome septique, et notamment un choc septique, peuvent être réanimés par une prise en charge standard, telle qu'un traitement antibiotique de large spectre mis en place avant les résultats des analyses bactériologiques indiquant la source infectieuse, d'autres patients, développant un syndrome septique beaucoup plus grave, nécessitent une mise en place de thérapeutiques lourdes, comme la protéine C activée. Au delà du coût très élevé, ce genre de thérapeutique expose les patients à des risques d'effets indésirables très importants (troubles de la coagulation...). Il est donc très important de cibler de manière efficace les patients susceptibles de bénéficier d'un tel traitement.

De ce fait, la connaissance de l'étiologie de la réponse systémique inflammatoire et la détermination de la susceptibilité du patient présentant une réponse systémique inflammatoire à contracter une infection nosocomiale sont essentiels pour proposer un traitement adapté au patient. Une autre donnée importante pour le patient est de pouvoir établir un pronostic le plus précoce possible d'évolution du choc septique.

A ce jour, il n'existe pas de test qui permette de déterminer la susceptibilité d'un patient à développer une infection nosocomiale, en particulier un patient présentant une réponse systémique inflammatoire liée ou non à une infection. Les inventeurs ont mis en évidence, de manière inattendue, que l'analyse de l'expression des gènes S100A9 et/ou S100A8 est particulièrement adaptée pour la détermination d'une telle susceptibilité et que de plus elle est appropriée pour l'établissement d'un pronostic d'évolution d'un choc septique.

Avant d'aller plus avant, les définitions suivantes sont données afin de mieux comprendre l'invention.

On entend par syndrome septique, une réponse systémique à l'infection. Ce syndrome septique peut être à un stade de SIRS, sepsis, sepsis sévère ou de choc septique. Préférentiellement, le syndrome septique est un choc septique.

On entend par infection nosocomiale, toute infection contractée dans un établissement de santé 48 heures après l'admission et qui n'était pas présente à l'admission du patient dans cet établissement.

On entend par gènes cibles le gène S100A9 et le gène S100A8, en particulier le gène S100A9 référencé dans GenBank sous le numéro d'accession NM_002965.3 et le gène S100A8 référencé dans GenBank sous le numéro d'accession NM_002964.3.

On entend par produit d'expression du gène S100A9 et du gène S100A8, l'ARN messager ou un fragment d'ARNm, l'ADNc ou un fragment d'ADNc, une protéine ou un fragment de protéine.

On entend par échantillon biologique, tout matériel issu du patient, susceptible de contenir un matériel biologique permettant de détecter l'expression d'un gène. Ce peut être notamment un échantillon de sang, de sérum, de salive ou de tissu ou de cellules circulantes du patient. On dispose de cet échantillon biologique par tout type de prélèvement connu de l'homme du métier, tel que notamment une prise de sang.

On entend par matériel biologique tout matériel permettant de détecter l'expression d'un gène, comme notamment un acide nucléique ou une protéine, ou sa séquence codante. L'acide nucléique peut être notamment un ARN (acide ribonucléique) tel qu'un ARNm (ARN messager). Selon un mode préféré de réalisation de l'invention, le matériel biologique est un acide nucléique, et encore plus préférentiellement un ARNm.

L'extraction du matériel biologique de l'échantillon biologique peut être réalisée par tous les protocoles d'extraction d'acides nucléiques ou de protéines bien connus de l'homme du métier.

A titre indicatif, l'extraction d'acides nucléique peut notamment être réalisée par :
- une étape de lyse des cellules présentes dans l'échantillon biologique, afin de libérer les acides nucléiques contenus dans les enveloppes protéiques et/ou lipidiques des microorganismes (comme des débris cellulaires qui perturbent les réactions ultérieures). A titre d'exemple, on peut utiliser les méthodes de lyse telles que décrite dans les demandes de brevet :
   o WO-A-00/05338 sur la lyse mixte magnétique et mécanique,
   o WO-A-99/53304 sur la lyse électrique, et
   o WO-A-99/15321 sur la lyse mécanique.

   L'homme du métier pourra utiliser d'autres méthodes de lyse bien connues, telles que les chocs thermiques ou osmotiques ou les lyses chimiques par des agents chaotropiques tels que les sels de guanidium (US-A-5,234,809).
- une étape de purification, permettant la séparation entre les acides nucléiques et les autres constituants cellulaires relargués dans l'étape de lyse. Cette étape permet généralement de concentrer les acides nucléiques. A titre d'exemple, on peut utiliser des particules magnétiques éventuellement revêtues d'oligonucléotides, par adsorption ou covalence (voir à ce sujet les brevets US-A-4,672,040 et US-A-5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet : WO-A-97/45202 et WO-A-99/35500. Un autre exemple intéressant de méthode de purification des acides nucléiques est l'utilisation de silice soit sous forme de colonne, soit sous forme de particules inertes^{[2]} ou magnétiques (Merck: MagPrep^{□} Silica, Promega: MagneSil™ Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind™). Une telle étape d'extraction peut être faite en automates. L'automate easyMAG^{®} est particulièrement adapté.

On entend par réactif spécifique un réactif qui réagit avec le matériel biologique afin de mettre en évidence, d'une manière directe ou indirecte l'expression d'un gène cible, qui peut être déterminé par l'analyse des ARNm issus de ce gène, ou par l'analyse de la protéine codée par ce gène.

A titre indicatif, lorsque l'on souhaite déterminer l'expression d'un gène cible par l'analyse de la protéine codée par ce gène, ce réactif spécifique comprend au moins un anticorps spécifique de la protéine codée par ce gène cible.

A titre indicatif, lorsque l'on souhaite déterminer l'expression d'un gène cible par l'analyse des ARNm transcrits à partir de ce gène, ce réactif spécifique comprend au moins une amorce d'amplification spécifique de l'ADN complémentaire de cet ARNm (on parle alors d'amorce d'amplification spécifique d'un gène cible). L'ADN complémentaire d'un ARNm peut être obtenu selon un protocole bien connu de l'homme du métier. A titre indicatif, on extrait les ARN totaux (comprenant les ARN ribosomaux, les ARN de transfert, les ARNm) de l'échantillon biologique. On réalise ensuite une réaction de transcription inverse à l'aide d'une enzyme transcriptase inverse qui permet d'obtenir, à partir d'un fragment d'ARN, un fragment complémentaire d'ADN (ADNc). La réalisation d'une telle étape est bien connue de l'homme du métier. Lorsque l'on souhaite plus particulièrement obtenir uniquement les ADN complémentaires des ARN messagers, on réalise cette étape enzymatique en présence de fragments nucléotidiques comprenant uniquement des bases thymine (polyT), qui s'hybrident par complémentarité sur la séquence polyA des différents ARNm afin de former un complexe polyT-polyA qui sert alors de point de départ à la réaction de transcription inverse réalisée par l'enzyme transcriptase inverse. On obtient alors différents ADN complémentaires des différents ARN messagers initialement présents dans l'échantillon biologique. Dans la suite de l'exposé, on désigne par ADNc, un ADN complémentaire d'un ARN messager.

Par amorce d'amplification, on entend un fragment nucléotidique comprenant de 5 à 100 motifs nucléotidiques, préférentiellement de 15 à 25 motifs nucléotidiques et possédant une spécificité d'hybridation dans des conditions déterminées pour l'initiation d'une polymérisation enzymatique, par exemple dans une réaction d'amplification enzymatique.

Par réaction d'amplification enzymatique, on entend un processus générant de multiples copies d'un fragment nucléotidique cible à l'aide d'amorces d'amplification spécifiques par l'action d'au moins une enzyme. De telles réactions d'amplification sont bien connues de l'homme du métier et on peut citer notamment les techniques suivantes : PCR (Polymerase Chain Reaction), LCR (Ligase Chain Reaction), RCR (Repair Chain Reaction), 3SR (Self Sustained Sequence Replication) avec la demande de brevet WO-A-90/06995, NASBA (Nucleic Acid Sequence-Based Amplification), et TMA (Transcription Mediated Amplification) avec le brevet US-A-5,399,491.

On parle alors d'amplicons pour désigner les polynucléotides générés par une technique d'amplification enzymatique. Préférentiellement, lorsque l'amplification enzymatique est une PCR, le réactif spécifique comprend au moins 2 amorces d'amplification spécifiques afin d'amplifier une région particulière de l'ADN complémentaire de l'ARNm issu du gène cible. Lorsque l'amplification enzymatique est une PCR réalisée après une réaction de transcription inverse, on parle de RT-PCR. Par sonde d'hybridation, on entend un fragment nucléotidique comprenant de 5 à 100 motifs nucléotidiques, notamment de 6 à 35 motifs nucléotidiques, possédant une spécificité d'hybridation dans des conditions déterminées pour former un complexe d'hybridation avec un fragment nucléotidique cible. Dans la présente invention, le fragment nucléotidique cible peut être une séquence nucléotidique comprise dans un ARN messager ou une séquence nucléotidique comprise dans un ADN complémentaire obtenu par transcription inverse dudit ARN messager.

Par hybridation, on entend le processus au cours duquel, dans des conditions appropriées, deux fragments nucléotidiques, tels que par exemple une sonde d'hybridation et un fragment nucléotidique cible, ayant des séquences suffisamment complémentaires sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier. En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Par détection on entend soit une détection directe par une méthode physique, soit une méthode de détection à l'aide d'un marqueur. De nombreuses méthodes de détection existent pour la détection des acides nucléiques^{[4,5]}.

Par marqueur, on entend un traceur capable d'engendrer un signal. Une liste non limitative de ces traceurs comprend les enzymes qui produisent un signal détectable par exemple par colorimétrie, fluorescence ou luminescence, comme la peroxydase de raifort, la phosphatase alcaline, la bêtagalactosidase, la glucose-6-phosphate déshydrogénase ; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I. Ainsi, le polynucléotide peut être marqué pendant l'étape d'amplification enzymatique, par exemple en utilisant un nucléotide triphosphate marqué pour la réaction d'amplification. Le nucléotide marqué sera un désoxyribonucléotide dans les systèmes d'amplification générant un ADN, comme la PCR, ou un ribonucléotide dans les techniques d'amplification générant un ARN, comme les techniques TMA ou NASBA. Le polynucléotide peut aussi être marqué après l'étape d'amplification, par exemple en hybridant une sonde marquée selon la technique d'hybridation sandwich décrite dans le document WO-A-91/19812.

Au sens de la présente invention, la sonde d'hybridation peut être une sonde dite de capture. Dans ce cas, le fragment nucléotidique cible peut être préalablement marqué au moyen d'un marqueur. La sonde dite de capture est immobilisée ou immobilisable sur un support solide par tout moyen approprié, c'est-à-dire directement ou indirectement, par exemple par covalence ou adsorption. On réalise alors une réaction d'hybridation entre ladite sonde de détection et le fragment nucléotidique cible marqué.

La sonde d'hybridation peut également être une sonde dite de détection. Dans ce cas, la sonde d'hybridation peut être marquée au moyen d'un marqueur. On réalise alors une réaction d'hybridation entre ladite sonde de capture et le fragment nucléotidique cible.

Que l'on utilise une sonde dite de capture ou une sonde dite de détection, la réaction d'hybridation peut être réalisée sur un support solide qui inclut tous les matériaux sur lesquels peut être immobilisé un acide nucléique. Des matériaux de synthèse ou des matériaux naturels, éventuellement modifiés chimiquement, peuvent être utilisés comme support solide, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex; des particules magnétiques ; des dérivés métalliques, des gels etc. Le support solide peut être sous la forme d'une plaque de microtitration, d'une membrane comme décrit dans la demande WO-A-94/12670, d'une particule ou d'une biopuce.

Dans la présente invention, la détermination de l'expression du gène S100A9 et du gène S100A8 peut être analysée par l'expression des ARNm qui sont transcrits à un temps donné. Dans ce cas, le matériel biologique est un acide nucléique, et le réactif spécifique peut être indifféremment une amorce d'amplification ou une sonde d'hybridation telles que définies précédemment.

On peut déterminer l'expression d'un gène cible de la manière suivante:
1) après avoir extrait les ARN totaux d'un échantillon biologique, on réalise une étape de transcription inverse, telle que décrite précédemment afin d'obtenir les différents ADN complémentaires des différents ARN messagers initialement présents dans l'échantillon biologique (ou ADNc)
2) on amplifie spécifiquement les ADNc. Dans ce cas, le réactif spécifique utilisé comprend au moins une amorce d'amplification spécifique du gène cible, telle que définie précédemment. Cette étape peut être réalisée notamment par une réaction d'amplification de type PCR ou par toute autre technique d'amplification appropriée.
3) on détermine l'expression du gène cible en quantifiant les ADNc. Les ADNc peuvent être quantifiés notamment par l'utilisation d'une gamme de quantification obtenue par une réaction d'amplification conduite jusqu'à saturation. Afin de tenir compte de la variabilité d'efficacité enzymatique qui peut être observée lors des différentes étapes (transcription inverse, PCR...), on peut normaliser l'expression du gène cible des différents groupes de patients, par la détermination simultanée de l'expression d'un gène dit de ménage, dont l'expression est similaire chez les différents groupes de patients. En réalisant un rapport entre l'expression du gène cible et l'expression du gène de ménage, on corrige ainsi toute variabilité entre les différentes expérimentations. L'homme du métier pourra se référer notamment aux publications suivantes [6,7].

On peut également déterminer l'expression d'un gène cible de la manière suivante:
1) après avoir extrait les ARN totaux d'un échantillon biologique, on réalise une étape de transcription inverse, telle que décrite précédemment afin d'obtenir les différents ADN complémentaires des différents ARN messagers initialement présents dans l'échantillon biologique (ou ADNc)
2) on immobilise les ADNc sur une membrane
3) on détermine l'expression du gène cible en hybridant les ADNc à des sondes d'hybridation spécifiques du gène cible préalablement marquées. De telles techniques d'hybridation sont bien connues de l'homme du métier, et on peut citer notamment la technique du Northern blot. Cette réaction d'hybridation peut être réalisée après une étape d'amplification spécifique des ADN complémentaires des ARN messagers d'un gène cible lorsque notamment le gène est faiblement exprimé.

L'expression d'un gène cible peut être également analysée par l'expression des protéines codées par le gène cible. Dans ce cas, le matériel biologique est une protéine et plusieurs techniques de détection avec ou sans ligand peuvent être utilisées. La spectrométrie de masse peut être utilisée comme technique de détection sans ligand. Le réactif spécifique peut être un anticorps spécifique de la protéine codée par le gène cible pour un système de détection avec ligand.

Les anticorps recombinants, spécifiques de la protéine traduite du gène cible peuvent être obtenus selon des procédés classiques connus de l'homme du métier, à partir d'organismes procaryotes, tels que bactéries, ou à partir d'organismes eucaryotes, tels que levures, cellules de mammifères, de plantes, d'insectes ou d'animaux, ou par des systèmes de production extra-cellulaire. Les anticorps monoclonaux peuvent être préparés selon les techniques classiques connues de l'homme du métier telles que la technique des hybridomes dont le principe général est rappelé ci-après.

Dans un premier temps, on immunise un animal, généralement une souris, (ou des cellules en culture dans le cadre d'immunisations *in vitro*) avec un antigène cible d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité.

Des fragments d'anticorps peuvent par exemple être obtenus par protéolyse. Ainsi, ils peuvent être obtenus par digestion enzymatique, résultant en des fragments de type Fab (traitement à la papaïne^{[8]}) ou de type F(ab)'2 (traitement à la pepsine^{[9]}) Ils peuvent également être préparés par voie recombinante^{[10]}. Un autre fragment d'anticorps qui convient aux fins de l'invention comprend un fragment Fv qui est un dimère constitué de l'association non covalente du domaine variable léger (VL) et du domaine variable lourd (VH) du fragment Fab, donc de l'association de deux chaînes polypeptidiques. Afin d'améliorer la stabilité du fragment Fv due à la dissociation des deux chaînes polypeptidiques, ce fragment Fv peut être modifié par génie génétique en insérant un lien peptidique adapté entre le domaine VL et le domaine VH^{[11]}. On parle alors de fragment scFv (« single chain Fragment variable ») car il est constitué d'une seule chaîne polypeptidique. L'utilisation d'un lien peptidique composé préférentiellement de 15 à 25 acides aminés permet de relier l'extrémité C-terminale d'un domaine à l'extrémité N-terminale de l'autre domaine, constituant ainsi une molécule monomérique dotée de propriétés de liaison similaires à celles de l'anticorps sous sa forme complète. Les deux orientations des domaines VL et VH conviennent (VL-lien-VH et VH-lien-VL) car elles présentent des propriétés fonctionnelles identiques. Bien entendu, tout fragment connu de l'homme du métier et présentant les caractéristiques immunologiques définies ci-dessus convient aux fins de l'invention.

Lorsque le matériel biologique est une protéine issue de l'expression d'un gène, on peut déterminer l'expression de ce dernier, en détectant et ou quantifiant la dite protéine par Western Blot ou ELISA, ou toute autre méthode connue de l'homme du métier, telle qu'une méthode de chimiluminescence à partir du matériel biologique.

La technique ELISA (« Enzyme Linked Immuno Sorbent Assay ») est un dosage immunoenzymatique sur support solide. Ce test entre dans le cadre plus général des EIA (« Enzyme Immunoassays ») dans lequel le dosage est couplé à une réaction catalysée par une enzyme. La technique utilise un ou deux anticorps. L'anticorps de détection de la formation de complexes immuns (antigène / anticorps) est couplé à l'enzyme et peut générer l'émission d'un signal par un substrat chromogène ou fluorogène.

La technique Western Blot est un test pour détecter une protéine spécifique dans un échantillon à l'aide d'un anticorps spécifique de cette protéine comprenant les étapes suivantes telle que décrites ci après.

La première étape est une électrophorèse sur gel, qui permet de séparer les protéines de l'échantillon selon leur taille.

Les protéines dans le gel sont alors transférées sur une membrane (nitrocellulose, PVDF...) par pression ou par application d'un courant électrique, les protéines se fixant à la membrane grâce à des interactions hydrophobes et ioniques.

Après saturation des sites d'interaction non spécifique, un premier anticorps, spécifique de la protéine à étudier (anticorps primaire) est incubé avec la membrane.

La membrane est ensuite rincée afin d'enlever les anticorps primaires non liés, puis incubée avec des anticorps dits secondaires, qui vont se lier aux anticorps primaires. Cet anticorps secondaire est habituellement lié à une enzyme qui permet l'identification visuelle de la protéine étudiée sur la membrane. L'ajout d'un substrat marqué de l'enzyme engendre une réaction colorée qui est visible sur la membrane.

L'analyse de l'expression du gène S100A9 et/ou du gène S100A8 permet de déterminer la susceptibilité d'un patient à contracter une infection nosocomiale, en particulier un patient présentant une réponse systémique inflammatoire, et d'établir un pronostic d'évolution d'un syndrome septique. On peut par exemple analyser l'expression d'au moins un gène cible chez un patient dont on ne connaît pas la susceptibilité à contracter une infection nosocomiale, et en comparant avec des valeurs d'expression moyenne connues du gène cible de patients sensibles et des valeurs d'expression moyenne connues du gène cible de patients non sensibles, déterminer si le patient est susceptible de contracter une infection nosocomiale. De la même manière, on peut par exemple analyser l'expression d'au moins un gène cible et en la comparant avec des moyennes d'expression connue du gène cible établir un pronostic d'évolution d'un syndrome septique, incluant un pronostic de survie ou de mort.

Il est décrit un procédé pour déterminer la susceptibilité à contracter une infection nosocomiale chez un patient selon lequel:
a) on dispose d'un échantillon biologique d'un patient et on extrait du matériel biologique de l'échantillon biologique
b) on dispose d'au moins un réactif spécifique d'un produit d'expression d'au moins un gène cible choisi parmi les gènes cibles S100A9 et S100A8
c) on détermine l'expression d'au moins un des gènes cibles S100A9 et S100A8, une surexpression par rapport à une valeur seuil déterminée étant une indication d'une susceptibilité à contracter une infection nosocomiale.

L'invention a en particulier pour objet un procédé pour déterminer la susceptibilité à contracter une infection nosocomiale chez un patient selon lequel :
a) on dispose d'un échantillon biologique d'un patient et on extrait du matériel biologique de l'échantillon biologique
b) on dispose d'au moins un réactif spécifique d'un produit d'expression d'au moins un gène cible qui est le gène cible S100A9,
c) on détermine l'expression du gène cible S100A9, une surexpression par rapport à une valeur seuil déterminée étant une indication d'une susceptibilité à contracter une infection nosocomiale.

On peut dans l'étape c) déterminer de plus l'expression du gène cible S100A8, une surexpression du gène cible S100A8 par rapport à une valeur seuil déterminée étant une indication d'une susceptibilité à contracter une infection nosocomiale.

L'homme du métier possèdent différentes possibilités pour déterminer une valeur seuil. On peut citer en exemple la détermination de l'indice de Youden.

L'indice de Youden correspond à une mesure du taux global de non erreur. Il varie entre 0, pour un test ne permettant pas la discrimination de deux populations, et 1, pour un test permettant une discrimination parfaite des deux populations. Le maximum de l'indice de Youden correspond à la valeur seuil pour laquelle le nombre de faux résultats (faux négatif ou faux positif) est le plus faible. Plus l'indice de Youden est proche de 1, plus les performances diagnostiques sont bonnes^{[12]}.

En particulier, l'échantillon biologique est prélevé chez un patient présentant une réponse systémique inflammatoire associée ou non à une infection. L'échantillon biologique peut être un échantillon de sang et le matériel biologique extrait peut être, entre autres, un acide nucléique. De préférence, on extrait les ARN totaux de l'échantillon biologique et la détermination de l'expression du gène cible est effectuée par l'analyse de l'expression des ARNm.

Dans un mode de réalisation préféré du procédé de l'invention le réactif spécifique comprend au moins une amorce d'amplification ou au moins une sonde d'hybridation spécifique du produit d'expression du gène cible S100A9 et/ou du gène cible S100A8 ; ou au moins un anticorps spécifique du produit d'expression du gène cible S100A9 et/ou du gène cible S100A8.

Le procédé est notamment appropriés pour déterminer la susceptibilité à contracter une infection nosocomiale chez un patient en syndrome septique, c'est à dire présentant une réponse systémique inflammatoire à une infection, tel que SIRS, sepsis, sepsis sévère et choc septique.

Le réactif spécifique d'un produit d'expression du gène S100A9 et/ou du gène S100A8 est donc utilisé pour déterminer la susceptibilité d'un patient à contracter une infection nosocomiale, en particulier un patient présentant une réponse systémique inflammatoire associée ou non à une infection. Le réactif peut comprendre au moins une sonde d'hybridation spécifique du gène cible S100A9, au moins une sonde d'hybridation spécifique du gène cible S100A8, au moins une amorce d'amplification spécifique du gène cible S100A9, au moins une amorce d'amplification spécifique du gène cible S100A8, au moins un anticorps spécifique de la molécule S100A9 ou moins un anticorps spécifique de la molécule S100A8.

L'analyse de l'expression du gène S100A9 et/ou S100A8 permet également d'établir un pronostic d'évolution d'un syndrome septique (pronostic incluant la survie et la mort).

C'est la raison pour laquelle un procédé pour l'établissement d'un pronostic de l'évolution d'un syndrome septique chez un patient selon ce procédé,
a) on dispose d'un échantillon biologique d'un patient et on extrait du matériel biologique de l'échantillon biologique
b) on dispose d'au moins un réactif spécifique d'un produit d'expression d'au moins un gène cible choisi parmi les gènes cibles S100A9 et S100A8
c) on détermine l'expression d'au moins un des gènes cibles S100A9 et S100A8, une sous-expression par rapport à une valeur seuil déterminée étant une indication d'un bon pronostic de l'évolution du syndrome septique et une surexpression par rapport à une valeur seuil déterminée étant une indication d'un mauvais pronostic de l'évolution du syndrome septique.

On peut à l'étape c) déterminer l'expression du gène cible S100A9 et du gène cible S100A8, une sous-expression du gène cible S100A9 par rapport à une valeur seuil déterminée et une sous-expression du gène cible S100A8 par rapport à une valeur seuil déterminée étant une indication d'un bon pronostic de l'évolution du syndrome septique ;
une surexpression du gène cible S100A9 par rapport à une valeur seuil déterminée et une surexpression du gène cible S100A8 par rapport à une valeur seuil déterminée par rapport à une valeur seuil déterminée étant une indication d'un mauvais pronostic d'évolution du syndrome septique.

Il est à la portée de l'homme du métier de déterminer une valeur seuil. On peut citer en exemple la détermination de l'indice de Youden. L'indice de Youden correspond à une mesure du taux global de non erreur. Il varie entre 0, pour un test ne permettant pas la discrimination de deux populations, et 1, pour un test permettant une discrimination parfaite des deux populations. Le maximum de l'indice de Youden correspond à la valeur seuil pour laquelle le nombre de faux résultats (faux négatif ou faux positif) est le plus faible. Plus l'indice de Youden est proche de 1, plus les performances diagnostiques sont bonnes^{[12]}.

L'échantillon biologique peut être un échantillon de sang et le matériel biologique extrait peut être, entre autres, un acide nucléique. De préférence, on extrait les ARN totaux de l'échantillon biologique et la détermination de l'expression du gène cible est effectuée par l'analyse de l'expression des ARNm. Dans un mode de réalisation préféré du procédé de l'invention le réactif spécifique comprend au moins une amorce d'amplification ou au moins une sonde d'hybridation spécifique du produit d'expression du gène cible S100A9 et/ou du gène cible S100A8 ; ou au moins un anticorps spécifique du produit d'expression du gène cible S100A9 et/ou du gène cible S100A8.

Le réactif spécifique d'un produit d'expression du gène S100A9 et/ou du gène S100A8 est donc utilisé pour établir un pronostic d'évolution d'un syndrome septique chez un patient (incluant un pronostic de survie ou de mort). Le réactif peut comprendre au moins une sonde d'hybridation spécifique du gène cible S100A9, au moins une sonde d'hybridation spécifique du gène cible S100A8, au moins une amorce d'amplification spécifique du gène cible S100A9, au moins une amorce d'amplification spécifique du gène cible S100A8, au moins un anticorps spécifique de la molécule S100A9 ou moins un anticorps spécifique de la molécule S100A8.

### FIGURES

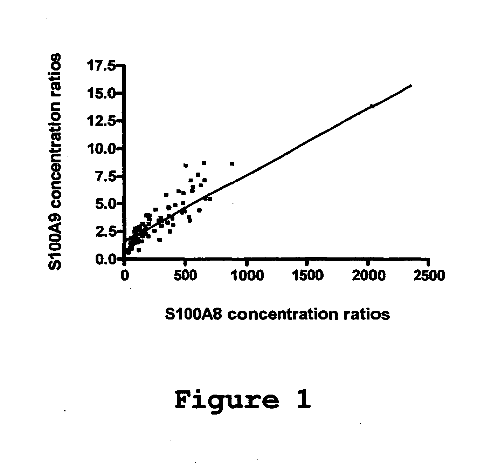

La figure 1 représente la corrélation de l'expression génique de S100A8 et S100A9 (n=86). Elle montre la forte corrélation qu'il existe entre les expressions géniques des molécules S100A9 et S100A8 avec un coefficient de Spearman > à 0,90 (r=0,9134). Les expressions géniques des molécules S100A9 et S100A8 possèdent une capacité similaire à discriminer les patients susceptibles de contracter une infection nosocomiale et à établir un pronostic d'évolution du syndrome septique.

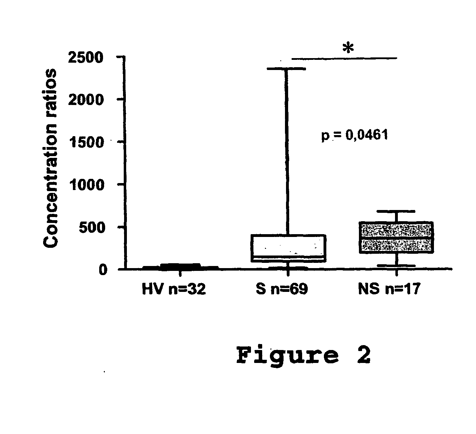

La figure 2 illustre la différence significative d'expression du gène S100A8 (p=0,0461) entre les patients survivants et non survivants sur des prélèvements réalisés entre J7 et J10 après le début du choc septique. Légende : HV = volontaires sains ; S = patients survivants ; NS = patients non survivants.

### EXEMPLES

### Exemple 1 - Etude de l'expression du gène S100A9

### Echantillons

Un groupe de 44 volontaires sains (S) et un groupe de 148 patients atteints d'un syndrome septique (SEP) et prélevés à J1, J2 ou J 3 après le choc septique (H0 étant l'injection du traitement vasopresseur) ont été utilisés, afin de comparer l'expression du gène S100A9 dans le sang périphérique. On distinguait alors un groupe de 44 patients sains (S), et un groupe de 148 patients développant un choc septique (SEP).

### Extraction des ARN et synthèse des ADNc

Pour chaque patient, l'échantillon biologique était un prélèvement sanguin, régulièrement obtenu pendant les 10 premiers jours suivant le début du syndrome septique développé par les patients SEP. Un prélèvement a également été effectué selon un même protocole chez les patients sains (S). Ces prélèvements ont été collecté directement dans des tubes PAXgeneTM Blood RNA (PreAnalytiX, Frankin Lakes, USA).

Après l'étape de prélèvement sanguin et afin d'obtenir une lyse totale des cellules, les tubes ont été laissés à température ambiante pendant 4 h puis conservés à -20°C jusqu'à l'extraction du matériel biologique. Plus précisément, dans ce protocole, les ARN totaux ont été extraits à l'aide des kits PAXgene Blood RNA® (PreAnalytiX) en respectant les recommandations du fabriquant. Brièvement, les tubes ont été centrifugés (10 min., 3000g) afin d'obtenir un culot d'acides nucléiques. Ce culot a été lavé et repris dans un tampon contenant de la protéinase K nécessaire à la digestion des protéines (10 min. à 55°C). Une nouvelle centrifugation (5 min. 19000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column et, avant l'élution de ceux-ci, une digestion de l'ADN contaminant a été effectuée à l'aide du RNAse free DNAse set™ (Qiagen Ltd, Crawley, UK).

Pour chaque extraction, la qualité des ARN totaux a été vérifiée par électrophorèse capillaire en utilisant le kit RNA 6000 Nano Chip™ (Agilent technologies). L'instrument utilisé est le bioanalyser 2100. Une réaction de transcription inverse (RT) a été réalisée dans un volume final de 20 µl. L'ARN total (0,5 µg) a été mélangé avec 1 µl de polyT à 50 µM et 1 µL d'annealing buffer puis incubé 5 min. à 65°C. Après refroidissement dans la glace, la solution a été mélangée avec 10 µl de 2x First Strand Reaction Mix et 2 µL de SuperScript III/RNase™ out enzyme Mix RT (15 U/µl), tous ces produits étant issus du SuperScript First Strand Synthesis Super Mix TM RT-PCR system (Invitrogen). La transcription inverse a été effectuée pendant 50 min. à 50°C puis stoppée par une incubation de 5 min. à 85°C. Pour finir, chaque solution d'ADNc a été diluée au 1/10^{ème} dans de l'eau DEPC.

### Préparation de gammes étalon de quantification

A partir d'un pool d'ADNc provenant de sujets sains, l'amplification de la région 153-179 du gène cible S100A9 (GenBank n° NM_002965.3) a été réalisée par PCR (Polymerase Chain Reaction), conduite jusqu'à saturation, en utilisant le couple d'amorces suivant :
Amorce sens : 5'-TCAAAGAGCTGGTGCGAAAA-3' (SEQ ID NO : 1)
Amorce anti-sens : 5'-AACTCCTCGAAGCTCAGCTG-3' (SEQ ID NO : 2)

### Analyse de l'expression des ARNm par PCR en temps réel

Les ARNm du gène cible S100A9 retranscrits en ADNc, comme décrit ci-dessus, ont été quantifiés par PCR quantitative en temps réel en utilisant le LightCycler™ (Roche). Les réactions PCR ont été effectuées à l'aide du Fast-Start™ DNA Master SYBR Green I real-time PCR kit (Roche Molecular Biochemicals). Chaque PCR a été effectuée dans un volume final de 20 µl contenant 3,6 µl d'eau, 2,4 µl de MgCl₂, 2 µl de SYBR Green mix (Fast start DNA master SYBR green + Taq DNA polymerase) et 1µl de chaque amorce (10 µM) et 10 µl de solution d'ADNc. Les amorces utilisées sont celles décrites précédemment.

Après une étape de dénaturation de 10 min. à 95°C, l'amplification est effectuée à l'aide de 40 cycles d'un protocole de "touch-down" PCR (10 sec. à 95°C, 10 sec. d'hybridation à 68-58°C, suivi d'une extension de 16 sec. à 72°C). A la fin de chaque cycle, la fluorescence émise par le SYBR Green a été mesurée.

Pour confirmer la spécificité de l'amplification, les produits PCR ont systématiquement fait l'objet d'une analyse de courbe de fusion (LightCycler™ - Roche). Pour cela, les produits PCR ont été traités par une température croissante de 58 à 98°C avec une augmentation de 0,1°C/sec. Pour chaque produit PCR, un seul pic a été obtenu lors de l'analyse de la courbe, caractérisé par une température de fusion spécifique.

La combinaison d'amorces nécessaires à la quantification du gène de ménage CPB a été fournie par Search-LC (Heidelberg, Allemagne; référence 488116).

La quantité d'ARNm cible relative à la quantité d'ARNm du gène de ménage cyclophilin B a été analysée par la technique de quantification relative avec le LightCycler Relative Quantification Software™ (Roche Molecular Biochemicals). La "Second Derivative Maximum Method" du logiciel LightCyclerTM (Roche) a été utilisée pour déterminer automatiquement le « Crossing Point » (Cp) pour chaque échantillon. La valeur du Cp a été définie comme le nombre de cycles pour lequel la fluorescence était significativement différente du bruit de fond.

Cinq dilutions en séries au 1/10^{ème} ont été réalisées en quadruplicate avec chaque standard afin de générer une courbe d'étalonnage exprimant le Cp en fonction du logarithme du nombre de copies. Les dilutions de standard ont été optimisées afin que la courbe d'étalonnage couvre le niveau d'expression attendu pour le gène cible et le gène de ménage. Les courbes standards relatives décrivant l'efficacité de PCR pour le gène cible et le gène de ménage ont été générées et utilisées pour réaliser une quantification avec le LightCycler Relative Quantification Software (Roche Molecular Biochemicals).

### Résultats obtenus

Les résultats obtenus sont présentés dans le tableau 1 ci-dessous.

**Tableau 1**

| **Patients** | **N** | **Mediane** | **Quartile inférieur** | **Quartile supérieur** |
|---|---|---|---|---|
| **S** | 44 | 1,46 | 1,065 | 1,87 |
| **SEP** | 148 | 16,62 | 11,87 | 25,835 |

La p_value du test de Mann-Whitney entre les sains et les patients atteints d'un choc septique est < 0,0001.

L'expression du marqueur S100A9 sur les 3 premiers jours du choc septique était significativement sur-exprimée par rapport aux donneurs sains.

### Exemple 2 - Etude de l'expression du gène S100A8

### Echantillons

Un groupe de 32 volontaires sains (S) et un groupe de 86 patients atteints d'un syndrome septique (SEP) et prélevés entre J7 et J10 après le choc septique (H0 étant l'injection du traitement vasopresseur) ont été utilisés, afin de comparer l'expression du gène S100A8 dans le sang périphérique. On distinguait alors un groupe de 32 patients sains (S), et un groupe de 86 patients développant un choc septique (SEP).

### Extraction des ARN et synthèse des ADNc

Pour chaque patient, l'échantillon biologique est un prélèvement sanguin, régulièrement obtenu pendant les 10 premiers jours suivant le début du syndrome septique développé par les patients SEP. Un prélèvement a également été effectué selon un même protocole chez les patients sains (S). Ces prélèvements ont été collecté directement dans des tubes PAXgeneTM Blood RNA (PreAnalytiX, Frankin Lakes, USA).

Après l'étape de prélèvement sanguin et afin d'obtenir une lyse totale des cellules, les tubes ont été laissés à température ambiante pendant 4 h puis conservés à -20°C jusqu'à l'extraction du matériel biologique. Plus précisément, dans ce protocole, les ARN totaux ont été extraits à l'aide des kits PAXgene Blood RNA® (PreAnalytiX) en respectant les recommandations du fabriquant. Brièvement, les tubes ont été centrifugés (10 min., 3000g) afin d'obtenir un culot d'acides nucléiques. Ce culot a été lavé et repris dans un tampon contenant de la protéinase K nécessaire à la digestion des protéines (10 min. à 55°C). Une nouvelle centrifugation (5 min. 19000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column et, avant l'élution de ceux-ci, une digestion de l'ADN contaminant a été effectuée à l'aide du RNAse free DNAse set™ (Qiagen Ltd, Crawley, UK).

Pour chaque extraction, la qualité des ARN totaux a été vérifiée par électrophorèse capillaire en utilisant le kit RNA 6000 Nano Chip™ (Agilent technologies). L'instrument utilisé est le bioanalyser 2100. Une réaction de transcription inverse (RT) a été réalisée dans un volume final de 20 µl. L'ARN total (0,5 µg) a été mélangé avec 1 µl de polyT à 50 µM et 1 µL d'annealing buffer puis incubé 5 min. à 65°C. Après refroidissement dans la glace, la solution a été mélangée avec 10 µl de 2x First Strand Reaction Mix et 2 µL de SuperScript III/RNase™ out enzyme Mix RT (15 U/µl), tous ces produits étant issus du SuperScript First Strand Synthesis Super Mix TM RT-PCR system (Invitrogen). La transcription inverse a été effectuée pendant 50 min. à 50°C puis stoppée par une incubation de 5 min. à 85°C. Pour finir, chaque solution d'ADNc a été diluée au 1/10^{ème} dans de l'eau DEPC.

### Préparation de gammes étalon de quantification

A partir d'un pool d'ADNc provenant de sujets sains, l'amplification de la région 129-280 du gène cible S100A8 (GenBank n° NM_002964.3) a été réalisée par PCR (Polymerase Chain Reaction), conduite jusqu'à saturation, en utilisant le couple d'amorces suivant :
Amorce sens : 5'-ATTTCCATGCCGTCTACAGG-3' (SEQ ID NO : 3)
Amorce anti-sens : 5'-CACCAGAATGAGGAACTCCT-3' (SEQ ID NO : 4)

### Analyse de l'expression des ARNm par PCR en temps réel

Les ARNm du gène cible S100A8 retranscrits en ADNc, comme décrit ci-dessus, ont été quantifiés par PCR quantitative en temps réel en utilisant le LightCycler™ (Roche). Les réactions PCR ont été effectuées à l'aide du Fast-Start™ DNA Master SYBR Green I real-time PCR kit (Roche Molecular Biochemicals). Chaque PCR a été effectuée dans un volume final de 20 µl contenant 3,6 µl d'eau, 2,4 µl de MgCl₂, 2 µl de SYBR Green mix (Fast start DNA master SYBR green + Taq DNA polymerase) et 1µl de chaque amorce (10 µM) et 10 µl de solution d'ADNc. Les amorces utilisées sont celles décrites précédemment.

Après une étape de dénaturation de 10 min. à 95°C, l'amplification est effectuée à l'aide de 40 cycles d'un protocole de "touch-down" PCR (10 sec. à 95°C, 10 sec. d'hybridation à 68-58°C, suivi d'une extension de 16 sec. à 72°C). A la fin de chaque cycle, la fluorescence émise par le SYBR Green a été mesurée.

Pour confirmer la spécificité de l'amplification, les produits PCR ont systématiquement fait l'objet d'une analyse de courbe de fusion (LightCycler™ - Roche). Pour cela, les produits PCR ont été traités par une température croissante de 58 à 98°C avec une augmentation de 0,1°C/sec. Pour chaque produit PCR, un seul pic a été obtenu lors de l'analyse de la courbe, caractérisé par une température de fusion spécifique.

La combinaison d'amorces nécessaires à la quantification du gène de ménage CPB a été fournie par Search-LC (Heidelberg, Allemagne; référence 488116).

La quantité d'ARNm cible relative à la quantité d'ARNm du gène de ménage cyclophilin B a été analysée par la technique de quantification relative avec le LightCycler Relative Quantification Software™ (Roche Molecular Biochemicals). La "Second Derivative Maximum Method" du logiciel LightCyclerTM (Roche) a été utilisée pour déterminer automatiquement le « Crossing Point » (Cp) pour chaque échantillon. La valeur du Cp a été définie comme le nombre de cycles pour lequel la fluorescence était significativement différente du bruit de fond.

Cinq dilutions en séries au 1/10^{ème} ont été réalisées en quadruplicate avec chaque standard afin de générer une courbe d'étalonnage exprimant le Cp en fonction du logarithme du nombre de copies. Les dilutions de standard ont été optimisées afin que la courbe d'étalonnage couvre le niveau d'expression attendu pour le gène cible et le gène de ménage. Les courbes standards relatives décrivant l'efficacité de PCR pour le gène cible et le gène de ménage ont été générées et utilisées pour réaliser une quantification avec le LightCycler Relative Quantification Software (Roche Molecular Biochemicals).

### Résultats obtenus

Les résultats obtenus sont présentés dans le tableau 2 ci-dessous.

**Tableau 2**

| **Patients** | **N** | **Mediane** | **Quartile inférieur** | **Quartile supérieur** |
|---|---|---|---|---|
| **S** | 32 | 12,9 | 8,5 | 20,4 |
| **SEP** | 86 | 183,5 | 96,6 | 438,5 |

La p_value du test de Mann-Whitney entre les sains et les patients atteints d'un choc septique est < 0,0001.

L'expression du marqueur S100A8 sur les jours J7 à J10 après le choc septique était significativement sur-exprimée par rapport aux donneurs sains.

### Exemple 3 - Etude de la valeur pronostic de survie de S100A8 chez des patients en choc septique.

### Echantillons

L'étude a été réalisée sur une cohorte de 86 patients ayant développé un choc septique, et admis dans un service de réanimation Chirurgicale ou Médicale du centre hospitalier Lyon-Sud. Ces patients ont été prélevés à J7, J8, J9 ou J10 après le choc septique (H0 étant le début du traitement vasopresseur).

### Extraction des ARN et synthèse des ADNc

Pour chaque patient, l'échantillon biologique est un prélèvement sanguin, qui a été prélevé quotidiennement pendant les 10 premiers jours suivant le début du syndrome septique (patients SEP). Ces prélèvements ont été collectés directement dans des tubes PAXgene™ Blood RNA (PreAnalytiX, Frankin Lakes, USA). Après l'étape de prélèvement sanguin et afin d'obtenir une lyse totale des cellules, les tubes ont été laissés à température ambiante pendant 4 heures puis conservés à -20°C jusqu'à l'extraction du matériel biologique. Plus précisément, dans ce protocole, les ARN totaux ont été extraits à l'aide des kits PAXgene Blood RNA® (PreAnalytiX) en respectant les recommandations du fabriquant. Brièvement, les tubes ont été centrifugés (10 min. à 3000g) afin d'obtenir un culot d'acides nucléiques. Ce culot a été lavé et repris dans un tampon contenant de la protéinase K nécessaire à la digestion des protéines (10 min. à 55°C). Une nouvelle centrifugation (5 min. à 19000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column™et, avant l'élution de ceux-ci, une digestion de l'ADN contaminant a été effectuée à l'aide du RNAse free DNAse set™ (Qiagen Ltd, Crawley, UK).

Pour chaque extraction, la qualité des ARN totaux a été vérifiée par électrophorèse capillaire en utilisant le kit RNA 6000 Nano Chip™ (Agilent technologies). L'instrument utilisé est le bioanalyser 2100™. Une réaction de transcription inverse (RT) a été réalisée dans un volume final de 20 µl. L'ARN total (0,5 µg) a été mélangé avec 1 µl de d'oligo (dT) 50 µM et 1 µL d'annealing buffer puis incubé 5 min. à 65°C. Après refroidissement dans la glace, la solution a été mélangée avec 10 µl de 2x First Strand Reaction Mix et 2 µL de SuperScript III/RNase out enzyme Mix RT (15 U/µl), tous ces produits étant issus du SuperScript First Strand Synthesis Super Mix™ RT-PCR system (Invitrogen). La transcription inverse a été effectuée pendant 50 min. à 50°C puis stoppée par une incubation de 5 min. à 85°C. Pour finir, chaque solution d'ADNc a été diluée au 1/10^{ème} dans de l'eau DEPC.

### Préparation de gammes étalon de quantification

A partir d'un pool d'ADNc provenant de sujets sains, l'amplification de la région 129-280 du gène cible S100A8 (GenBank n° NM_002964.3 ) a été réalisée par PCR (Polymerase Chain Reaction), conduite jusqu'à saturation, en utilisant le couple d'amorces suivant :
Amorce sens : 5'-ATTTCCATGCCGTCTACAGG-3' (SEQ ID NO : 3)
Amorce anti-sens : 5'-CACCAGAATGAGGAACTCCT-3' (SEQ ID NO : 4)

### Analyse de l'expression des ARNm par PCR quantitative en temps réel

Les ARNm du gène cible S100A8 retranscrits en ADNc, comme décrit ci-dessus, ont été quantifiés par PCR quantitative en temps réel en utilisant le LightCycler™ 2.0(Roche). Les réactions PCR ont été effectuées à l'aide du Fast-Start™ DNA Master SYBR Green I real-time PCR kit (Roche Molecular Biochemicals). Chaque PCR a été effectuée dans un volume final de 20 µl contenant 3,6 µl d'eau, 2,4 µl de MgCl₂, 2 µl de SYBR Green mix (Fast start DNA master SYBR green + Taq DNA polymerase) et 1µl de chaque amorce (10 µM) et 10 µl de solution d'ADNc. Les amorces utilisées sont celles décrites précédemment.

Après une étape de dénaturation de 10 min. à 95°C, l'amplification est effectuée à l'aide de 40 cycles d'un protocole de "touch-down" PCR (10 sec. à 95°C, 10 sec. d'hybridation à 68-58°C (0.5°C/cycle), suivi d'une extension de 16 sec. à 72°C). A la fin de chaque cycle, la fluorescence émise par le SYBR Green a été mesurée.

Pour confirmer la spécificité de l'amplification, les produits PCR ont systématiquement fait l'objet d'une analyse de courbe de fusion (LightCycler™ 2.0 - Roche). Pour cela, les produits PCR ont été traités par une température croissante de 58 à 98°C avec une augmentation de 0,1°C/sec. Pour chaque produit PCR, un seul pic a été obtenu lors de l'analyse de la courbe, caractérisé par une température de fusion spécifique.

La combinaison d'amorces nécessaires à la quantification du gène de ménage CPB a été fournie par Search-LC (Heidelberg, Allemagne; référence 488116).

La quantité d'ARNm cible relative à la quantité d'ARNm du gène de ménage cyclophilin B a été analysée par la technique de quantification relative avec le LightCycler Relative Quantification Software™ (Roche Molecular Biochemicals). La "Second Derivative Maximum Method" du logiciel LightCyclerTM (Roche) a été utilisée pour déterminer automatiquement le « Crossing Point » (Cp) pour chaque échantillon. La valeur du Cp a été définie comme le nombre de cycles pour lequel la fluorescence était significativement différente du bruit de fond.

Cinq dilutions en séries au 1/10^{ème} ont été réalisées en quadruplicate avec chaque standard afin de générer une courbe d'étalonnage exprimant le Cp en fonction du logarithme du nombre de copies. Les dilutions de standard ont été optimisées afin que la courbe d'étalonnage couvre le niveau d'expression attendu pour le gène cible et le gène de ménage. Les courbes standards relatives décrivant l'efficacité de PCR pour le gène cible et le gène de ménage ont été générées et utilisées pour réaliser une quantification avec le LightCycler Relative Quantification Software (Roche Molecular Biochemicals).

### Analyse des résultats

Afin de déterminer si l'expression du gène S100A8 est corrélée avec la survie du patient, un test de Mann Whitney a été fait.

La figure 2 illustre la différence significative d'expression du gène S100A8 (p=0,0461) entre les patients survivants et non survivants sur des prélèvements réalisés entre J7 et J10 après le début du choc septique.

### Exemple 4 - Etude de l'expression du gène S100A9 pour déterminer la susceptibilité à contracter une infection nosocomiale de patients

### Echantillons

L'étude a été réalisée sur une cohorte de 93 patients ayant développé un choc septique, et admis dans un service de réanimation Chirurgicale ou Médicale du centre hospitalier Lyon-Sud. Ces patients ont été prélevés à J7, J8, J9 ou J10 après le choc septique (H0 étant le début du traitement vasopresseur). Parmi ces patients ayant développé un choc septique 27 patients ont contracté secondairement au choc une infection nosocomiale et 66 n'ont pas contracté de infection nosocomiale dans les 53 jours d'observation de cette cohorte.

### Extraction des ARN et synthèse des ADNc

Pour chaque patient, l'échantillon biologique est un prélèvement sanguin, qui a été régulièrement obtenu pendant les 10 premiers jours suivant le début du syndrome septique (patients SEP). Un prélèvement a également été effectué selon un même protocole chez les sujets sains (S). Ces prélèvements ont été collecté directement dans des tubes PAXgeneTM Blood RNA (PreAnalytiX, Frankin Lakes, USA).

Après l'étape de prélèvement sanguin et afin d'obtenir une lyse totale des cellules, les tubes ont été laissés à température ambiante pendant 4 heures puis conservés à -20°C jusqu'à l'extraction du matériel biologique. Plus précisément, dans ce protocole, les ARN totaux ont été extraits à l'aide des kits PAXgene Blood RNA® (PreAnalytiX) en respectant les recommandations du fabriquant. Brièvement, les tubes ont été centrifugés (10 min. à 3000g) afin d'obtenir un culot d'acides nucléiques. Ce culot a été lavé et repris dans un tampon contenant de la protéinase K nécessaire à la digestion des protéines (10 min. à 55°C). Une nouvelle centrifugation (5 min. à 19000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column™ et, avant l'élution de ceux-ci, une digestion de l'ADN contaminant a été effectuée à l'aide du RNAse free DNAse set™ (Qiagen Ltd, Crawley, UK).

Pour chaque extraction, la qualité des ARN totaux a été vérifiée par électrophorèse capillaire en utilisant le kit RNA 6000 Nano Chip™ (Agilent technologies). L'instrument utilisé est le bioanalyser 2100™. Une réaction de transcription inverse (RT) a été réalisée dans un volume final de 20 µl. L'ARN total (0,5 µg) a été mélangé avec 1 µl de polyT à 50 µM et 1 µl de d'oligo (dT) 50 µM et 1 µL d'annealing buffer puis incubé 5 min. à 65°C. Après refroidissement dans la glace, la solution a été mélangée avec 10 µl de 2x First Strand Reaction Mix et 2 µL de SuperScript III/RNase out enzyme Mix RT (15 U/µl), tous ces produits étant issus du SuperScript First Strand Synthesis Super Mix™ RT-PCR system (Invitrogen). La transcription inverse a été effectuée pendant 50 min. à 50°C puis stoppée par une incubation de 5 min. à 85°C. Pour finir, chaque solution d'ADNc a été diluée au 1/10^{ème} dans de l'eau DEPC.

### Préparation de gammes étalon de quantification

A partir d'un pool d'ADNc provenant de sujets sains, l'amplification de la région 153-179 du gène cible S100A9 (GenBank n° NM_002965.3) a été réalisée par PCR (Polymerase Chain Reaction), conduite jusqu'à saturation, en utilisant le couple d'amorces suivant :
Amorce sens : 5'-TCAAAGAGCTGGTGCGAAAA-3' (SEQ ID NO : 1)
Amorce anti-sens : 5'-AACTCCTCGAAGCTCAGCTG-3' (SEQ ID NO : 2)

### Analyse de l'expression des ARNm par PCR en temps réel

Les ARNm du gène cible S100A9 retranscrits en ADNc, comme décrit ci-dessus, ont été quantifiés par PCR quantitative en temps réel en utilisant le LightCycler™ (Roche). Les réactions PCR ont été effectuées à l'aide du Fast-Start™ DNA Master SYBR Green I real-time PCR kit (Roche Molecular Biochemicals). Chaque PCR a été effectuée dans un volume final de 20 µl contenant 3,6 µl d'eau, 2,4 µl de MgCl₂, 2 µl de SYBR Green mix (Fast start DNA master SYBR green + Taq DNA polymerase) et 1µl de chaque amorce (10 µM) et 10 µl de solution d'ADNc. Les amorces utilisées sont celles décrites précédemment.

Après une étape de dénaturation de 10 min. à 95°C, l'amplification est effectuée à l'aide de 40 cycles d'un protocole de "touch-down" PCR (10 sec. à 95°C, 10 sec. d'hybridation à 68-58°C, suivi d'une extension de 16 sec. à 72°C). A la fin de chaque cycle, la fluorescence émise par le SYBR Green a été mesurée.

Pour confirmer la spécificité de l'amplification, les produits PCR ont systématiquement fait l'objet d'une analyse de courbe de fusion (LightCycler™ - Roche). Pour cela, les produits PCR ont été traités par une température croissante de 58 à 98°C avec une augmentation de 0,1°C/sec. Pour chaque produit PCR, un seul pic a été obtenu lors de l'analyse de la courbe, caractérisé par une température de fusion spécifique.

La combinaison d'amorces nécessaires à la quantification du gène de ménage CPB a été fournie par Search-LC (Heidelberg, Allemagne; référence 488116).

La quantité d'ARNm cible relative à la quantité d'ARNm du gène de ménage cyclophilin B a été analysée par la technique de quantification relative avec le LightCycler Relative Quantification Software™ (Roche Molecular Biochemicals). La "Second Derivative Maximum Method" du logiciel LightCyclerTM (Roche) a été utilisée pour déterminer automatiquement le « Crossing Point » (Cp) pour chaque échantillon. La valeur du Cp a été définie comme le nombre de cycles pour lequel la fluorescence était significativement différente du bruit de fond.

Cinq dilutions en séries au 1/10^{ème} ont été réalisées en quadruplicate avec chaque standard afin de générer une courbe d'étalonnage exprimant le Cp en fonction du logarithme du nombre de copies. Les dilutions de standard ont été optimisées afin que la courbe d'étalonnage couvre le niveau d'expression attendu pour le gène cible et le gène de ménage. Les courbes standards relatives décrivant l'efficacité de PCR pour le gène cible et le gène de ménage ont été générées et utilisées pour réaliser une quantification avec le LightCycler Relative Quantification Software (Roche Molecular Biochemicals).

### Résultats des analyses univariée et multivariée

Afin de déterminer les variables influant sur la survenue d'une infection nosocomiale, une analyse univariée puis une analyse multivariée sont réalisées. L'analyse univariée est réalisée en effectuant une régression logistique du statut atteint ou non atteint d'une infection nosocomiale en fonction de différentes variables et sur le marqueur S100A9 en ARNm sur les jours J7 à J10 après le début du choc septique. Ensuite une régression logistique multivariée est réalisée sur les variables présentant un niveau de significativité (p) <0,15 avec l'analyse univariée. La méthode de sélection « backward » (au départ toutes les variables sont prises en compte dans le modèle, puis elles sont retirées une à une) est utilisée avec un seuil de significativité à 0,05. Les résultats sont présentés dans le tableau 3 qui suit. L'odd ratio (O.R.) et son intervalle de confiance à 95% sont indiqués pour chaque résultat.

**Tableau 3**

| | | Analyse univariée (N=93) | | | Analyse multivariée (N=93) | | |
|---|---|---|---|---|---|---|---|
| | | OR | 95% CI | p | OR | 95% CI | p |
| Sexe | Masculin | 1 | - | - | | | |
| | Féminin | 1.6 | 0.641-3.997 | 0.3143 | | | |
| Age (ans) | > 65 | 1 | - | - | - | - | - |
| | ≤ 65 | 2.04 | 0.814-5.115 | 0.1285 | - | - | 0.1186 |
| SAPS II à l'admission | | | | | | | |
| | > 51 | 1 | - | - | | | |
| | ≤ 51 | 1.107 | 0.450-2.723 | 0.8246 | | | |
| SOFA à l'admission | | | | | | | |
| | < 10 | 1 | - | - | - | - | - |
| | ≥ 10 | 3.036 | 1.132-8.144 | 0.0274 | - | - | 0.1384 |
| Co-morbiditées | 0 | 1 | - | - | | | |
| | ≥ 1 | 1.021 | 0.414-2.514 | 0.9645 | | | |
| Type d'infection | | | | | | | |
| | Nosocomiale | 1 | - | - | | | |
| | Communautaire | 1.144 | 0.467-2.803 | 0.7682 | | | |
| Site d'infection | | | | | | | |
| | Pulmonaire | 1 | - | - | | | |
| | Abdominale | 1.735 | 0.644-4.672 | 0.3268 | | | |
| | Autres | 1.172 | 0.299-4.597 | 0.8540 | | | |
| S100A9 (mRNA) | < 6.1 | 1 | - | - | 1 | - | - |
| | ≥ 6.1 | 6.27 | 1.718-22.891 | 0.0055 | 6.242 | 1.662-23.447 | 0.0067 |

| | | | | | | | |
|---|---|---|---|---|---|---|---|
| OR : Odd Ratio CI : intervalle de confiance P : seuil de significativité | | | | | | | |

Comme cela ressort du tableau 3 l'analyse multivariée révèle qu'une expression du marqueur S100A9 ≥ 6,1 sur les jours 7 à 10 après le début du choc septique augmentent significativement la probabilité de survenue d'une infection nosocomiale (odd ratio à 6, 2).

### Exemple 5 - Etude de corrélation de l'expression du gène S100A9 et de l'expression du gène S100A8.

### Echantillons

L'étude a été réalisée sur une cohorte de 86 patients ayant développé un choc septique, et admis dans un service de réanimation Chirurgicale ou Médicale du centre hospitalier Lyon-Sud. Ces patients ont été prélevés à J7, J8, J9 ou J10 après le choc septique (H0 étant le début du traitement vasopresseur).

### Extraction des ARN et synthèse des ADNc

Pour chaque patient, l'échantillon biologique est un prélèvement sanguin, qui a été prélevé quotidiennement pendant les 10 premiers jours suivant le début du syndrome septique (patients SEP). Ces prélèvements ont été collectés directement dans des tubes PAXgene™ Blood RNA (PreAnalytiX, Frankin Lakes, USA). Après l'étape de prélèvement sanguin et afin d'obtenir une lyse totale des cellules, les tubes ont été laissés à température ambiante pendant 4 heures puis conservés à -20°C jusqu'à l'extraction du matériel biologique. Plus précisément, dans ce protocole, les ARN totaux ont été extraits à l'aide des kits PAXgene Blood RNA® (PreAnalytiX) en respectant les recommandations du fabriquant. Brièvement, les tubes ont été centrifugés (10 min. à 3000g) afin d'obtenir un culot d'acides nucléiques. Ce culot a été lavé et repris dans un tampon contenant de la protéinase K nécessaire à la digestion des protéines (10 min. à 55°C). Une nouvelle centrifugation (5 min. à 19000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column™et, avant l'élution de ceux-ci, une digestion de l'ADN contaminant a été effectuée à l'aide du RNAse free DNAse set™ (Qiagen Ltd, Crawley, UK).

Pour chaque extraction, la qualité des ARN totaux a été vérifiée par électrophorèse capillaire en utilisant le kit RNA 6000 Nano Chip™ (Agilent technologies). L'instrument utilisé est le bioanalyser 2100™. Une réaction de transcription inverse (RT) a été réalisée dans un volume final de 20 µl. L'ARN total (0,5 µg) a été mélangé avec 1 µl de d'oligo (dT) 50 µM et 1 µL d'annealing buffer puis incubé 5 min. à 65°C. Après refroidissement dans la glace, la solution a été mélangée avec 10 µl de 2x First Strand Reaction Mix et 2 µL de SuperScript III/RNase out enzyme Mix RT (15 U/µl), tous ces produits étant issus du SuperScript First Strand Synthesis Super Mix™ RT-PCR system (Invitrogen). La transcription inverse a été effectuée pendant 50 min. à 50°C puis stoppée par une incubation de 5 min. à 85°C. Pour finir, chaque solution d'ADNc a été diluée au 1/10^{ème} dans de l'eau DEPC.

### Préparation de gammes étalon de quantification

A partir d'un pool d'ADNc provenant de sujets sains, l'amplification de la région 153-179 du gène cible S100A9 (GenBank n° NM_002965.3) a été réalisée par PCR (Polymerase Chain Reaction), conduite jusqu'à saturation, en utilisant le couple d'amorces suivant :
Amorce sens : 5'-TCAAAGAGCTGGTGCGAAAA-3' (SEQ ID NO : 1)
Amorce anti-sens : 5'-AACTCCTCGAAGCTCAGCTG-3' (SEQ ID NO : 2)

A partir d'un pool d'ADNc provenant de sujets sains, l'amplification de la région 129-280 du gène cible S100A8 (GenBank n° NM_002964.3) a été réalisée par PCR (Polymerase Chain Reaction), conduite jusqu'à saturation, en utilisant le couple d'amorces suivant :
Amorce sens : 5'-ATTTCCATGCCGTCTACAGG-3' (SEQ ID NO : 3)
Amorce anti-sens : 5'-CACCAGAATGAGGAACTCCT-3' (SEQ ID NO : 4)

### Analyse de l'expression des ARNm par PCR quantitative en temps réel

Les ARNm des gènes cibles S100A9 et S100A8 retranscrits en ADNc, comme décrit ci-dessus, ont été quantifiés par PCR quantitative en temps réel en utilisant le LightCycler™ 2.0(Roche). Les réactions PCR ont été effectuées à l'aide du Fast-Start™ DNA Master SYBR Green I real-time PCR kit (Roche Molecular Biochemicals). Chaque PCR a été effectuée dans un volume final de 20 µl contenant 3,6 µl d'eau, 2,4 µl de MgCl₂, 2 µl de SYBR Green mix (Fast start DNA master SYBR green + Taq DNA polymerase) et 1µl de chaque amorce (10 µM) et 10 µl de solution d'ADNc. Les amorces utilisées sont celles décrites précédemment. Après une étape de dénaturation de 10 min. à 95°C, l'amplification est effectuée à l'aide de 40 cycles d'un protocole de "touch-down" PCR (10 s à 95°C, 10 s d'hybridation à 68-58°C (0.5°C/cycle), suivi d'une extension de 16 s à 72°C). A la fin de chaque cycle, la fluorescence émise par le SYBR Green a été mesurée.

Pour confirmer la spécificité de l'amplification, les produits PCR ont systématiquement fait l'objet d'une analyse de courbe de fusion (LightCycler™ 2.0 - Roche). Pour cela, les produits PCR ont été traités par une température croissante de 58 à 98°C avec une augmentation de 0,1°C/s. Pour chaque produit PCR, un seul pic a été obtenu lors de l'analyse de la courbe, caractérisé par une température de fusion spécifique.

La combinaison d'amorces nécessaires à la quantification du gène de ménage CPB a été fournie par Search-LC (Heidelberg, Allemagne; référence 488116).

La quantité des ARNm cibles relative à la quantité d'ARNm du gène de ménage cyclophilin B a été analysée par la technique de quantification relative avec le LightCycler Relative Quantification Software™ (Roche Molecular Biochemicals). La "Second Derivative Maximum Method" du logiciel LightCyclerTM (Roche) a été utilisée pour déterminer automatiquement le « Crossing Point » (Cp) pour chaque échantillon. La valeur du Cp a été définie comme le nombre de cycles pour lequel la fluorescence était significativement différente du bruit de fond.

Cinq dilutions en séries au 1/10^{ème} ont été réalisées en quadruplicate avec chaque standard afin de générer une courbe d'étalonnage exprimant le Cp en fonction du logarithme du nombre de copies. Les dilutions de standard ont été optimisées afin que les courbes d'étalonnage couvrent le niveau d'expression attendu pour les gènes cibles et le gène de ménage. Les courbes standards relatives décrivant l'efficacité de PCR pour le gène cible et le gène de ménage ont été générées et utilisées pour réaliser une quantification avec le LightCycler Relative Quantification Software (Roche Molecular Biochemicals).

### Analyse des résultats

Afin de déterminer si l'expression du gène S100A9 est corrélée avec l'expression du gène S100A8, une analyse de corrélation a été réalisée. Les résultats présentés à la figure 1 montrent qu'il existe une forte corrélation entre les expressions géniques de ces deux molécules, avec un coefficient de Spearman > à 0.90. Les expressions géniques des molécules S100A9 et S100A8 possèdent donc une capacité similaire à discriminer les patients susceptibles de contracter une infection nosocomiale et à établir un pronostic d'évolution d'un syndrome septique.

### Références bibliographiques

[1] M.M. Levy, M.P. Fink, J.C. Marshall, E. Abraham, D. Angus, D. Cook, J. Cohen, S.M. Opal, J.L. Vincent and G. Ramsay, 2001 SCCM/ESICM/ACCP/ATS/SIS International Sepsis Definitions Conference, Crit. Care Med. 31 (2003), pp. 1250-1256
[2] Boom R. et al., J. Clin. Microbiol., 1990, n°28(3), p. 495-503
[4] Kricka et al., Clinical Chemistry, 1999, n° 45(4), p.453-458
[5] Keller G.H. et al., DNA Probes, 2nd Ed., Stockton Press, 1993, sections 5 et 6, p.173-249
[6] Bustin SA Journal of molecular endocrinology, 2002, 29 : 23-39
[7] Giulietti A Methods, 2001, 25 : 386-401
[8] Porter RR, 1959, Biochem. J., 73 : 119-126
[9] Nisonoff A. et al., 1960, Science, 132 : 1770-1771
[10] Skerra A., 1993, Curr. Opin. Immunol., 5 : 256-262
[11] Huston P. et al., 1988, Proc. Natl.Acad. Sci.USA, 85 : 5879-5883
[12] Fluss R., Faraggi D., Reiser B. (2005), "Estimation of the Youden index and its associated cutoff point". Biometrical Journal,47:458-72

### SEQUENCE LISTING

<110> bioMérieux HOSPICES CIVILS DE LYON
<120> Procédés pour déterminer la susceptibilité à contracter une infection nosocomiale chez un patient et pour établir un pronostic d'évolution d'un syndrome septique
<130> S100
<160> 4
<170> **PatentIn version 3.3**
<210> 1
   <211> 20
   <212> DNA
   <213> Artificial
<220>
   <223> Amorce 1
<400> 1
   tcaaagagct ggtgcgaaaa 20
<210> 2
   <211> 20
   <212> DNA
   <213> Artificial
<220>
   <223> Amorce 2
<400> 2
   aactcctcga agctcagctg 20
<210> 3
   <211> 20
   <212> DNA
   <213> Artificial
<220>
   <223> S100A8
<400> 3
   atttccatgc cgtctacagg 20
<210> 4
   <211> 20
   <212> DNA
   <213> Artificial
<220>
   <223> S100A8
400> 4
   caccagaatg aggaactcct 20

## Revendications

1. Procédé pour déterminer la susceptibilité d'un patient à contracter une infection nosocomiale dans lequel:
a) on dispose d'un échantillon biologique d'un patient et on extrait du matériel biologique de l'échantillon biologique
b) on dispose d'au moins un réactif spécifique d'un produit d'expression d'au moins un gène cible qui est le gène cible S100A9
c) on détermine l'expression du gène cible S100A9, une surexpression par rapport à une valeur seuil déterminée étant une indication d'une susceptibilité à contracter une infection nosocomiale.

2. Procédé selon la revendication 1, dans lequel à l'étape c) on détermine de plus l'expression du gène cible S100A8, une surexpression du gène cible S100A8 par rapport à une valeur seuil déterminée étant une indication d'une susceptibilité à contracter une infection nosocomiale.

3. Procédé selon la revendication 1 ou 2, dans lequel l'échantillon biologique a été prélevé chez un patient présentant une réponse systémique inflammatoire associée ou non à une infection.

4. Procédé selon la revendication 1 ou 2, dans lequel l'échantillon biologique est un échantillon sanguin.

5. Procédé selon la revendication 1 ou 2 dans lequel le matériel biologique est un acide nucléique ou une protéine.

6. Procédé selon l'une quelconque des revendications 1 à 5, dans lequel le réactif spécifique du produit d'expression du gène S100A9 comprend au moins une amorce d'amplification spécifique du gène cible S100A9.

7. Procédé selon l'une quelconque des revendications 2 à 5, dans lequel le réactif spécifique du produit d'expression du gène S100A8 comprend au moins une amorce d'amplification spécifique du gène cible S100A8.

8. Procédé selon l'une quelconque des revendications 1 à 5, dans lequel le réactif spécifique du produit d'expression du gène S100A9 comprend au moins une sonde d'hybridation spécifique du gène cible S100A9.

9. Procédé selon l'une quelconque des revendications 2 à 5, dans lequel le réactif spécifique du produit d'expression du gène S100A8 comprend au moins une sonde d'hybridation spécifique du gène cible S100A8.

10. Procédé selon l'une quelconque des revendications 1 à 5, dans lequel le réactif spécifique du produit d'expression du gène S100A9 comprend au moins un anticorps spécifique de la molécule S100A9.

11. Procédé selon l'une quelconque des revendications 2 à 5, dans lequel le réactif spécifique du produit d'expression du gène S100A8 comprend au moins un anticorps spécifique de la molécule S100A8.

## Patentansprüche

1. Verfahren zur Bestimmung der Anfälligkeit eines Patienten für eine nosokomiale Infektion, wobei:
a) man über eine biologische Probe eines Patienten verfügt und aus der biologischen Probe biologisches Material entnimmt;
b) man über mindestens ein Reagenz mit Spezifität für ein Expressionsprodukt mindestens eines Zielgens, bei dem es sich um das S100A9-Zielgen handelt, verfügt,
c) man die Expression des S100A9-Zielgens bestimmt, wobei eine Überexpression in Bezug auf einen bestimmten Schwellenwert eine Anfälligkeit für eine nosokomiale Infektion anzeigt.

2. Verfahren nach Anspruch 1, wobei man in Schritt c) weiterhin die Expression des S100A8-Zielgens bestimmt, wobei eine Überexpression des S100A8-Zielgens im Vergleich zu einem bestimmten Schwellenwert eine Anfälligkeit für eine nosokomiale Infektion anzeigt.

3. Verfahren nach Anspruch 1 oder 2, wobei die biologische Probe von einem Patienten entnommen wurde, der eine gegebenenfalls mit einer Infektion assoziierte systemische Entzündungsreaktion anzeigt.

4. Verfahren nach Anspruch 1 oder 2, wobei es sich bei der biologischen Probe um eine Blutprobe handelt.

5. Verfahren nach Anspruch 1 oder 2, wobei es sich bei dem biologischen Material um eine Nukleinsäure oder ein Protein handelt.

6. Verfahren nach einem der Ansprüche 1 bis 5, wobei das Reagenz mit Spezifität für das Expressionsprodukt des S100A9-Gens mindestens einen für das S100A9-Zielgen spezifischen Amplifikationsprimer umfasst.

7. Verfahren nach einem der Ansprüche 2 bis 5, wobei das Reagenz mit Spezifität für das Expressionsprodukt des S100A8-Gens mindestens einen für das S100A8-Zielgen spezifischen Amplifikationsprimer umfasst.

8. Verfahren nach einem der Ansprüche 1 bis 5, wobei das Reagenz mit Spezifität für das Expressionsprodukt des S100A9-Gens mindestens eine für das S100A9-Zielgen spezifische Hydridisierungssonde umfasst.

9. Verfahren nach einem der Ansprüche 2 bis 5, wobei das Reagenz mit Spezifität für das Expressionsprodukt des S100A8-Gens mindestens eine für das S100A8-Zielgen spezifische Hydridisierungssonde umfasst.

10. Verfahren nach einem der Ansprüche 1 bis 5, wobei das Reagenz mit Spezifität für das Expressionsprodukt des S100A9-Gens mindestens einen für das S100A9-Molekül spezifischen Antikörper umfasst.

11. Verfahren nach einem der Ansprüche 2 bis 5, wobei das Reagenz mit Spezifität für das Expressionsprodukt des S100A8-Gens mindestens einen für das S100A8-Molekül spezifischen Antikörper umfasst.

## Claims

1. Method for determining a patient's susceptibility of contracting a nosocomial infection in which:
a) a biological sample is obtained from a patient and the biological material is extracted from the biological sample
b) at least one specific reagent of an expression product of at least one target gene which is the S100A9 target gene is prepared
c) the expression of the target gene S100A9 is determined, overexpression relative to a specified threshold value being an indication of susceptibility of contracting a nosocomial infection.

2. Method according to Claim 1, in which the expression of the S100A8 target gene is also determined in stage c), overexpression of the S100A8 target gene relative to a specified threshold value being an indication of susceptibility of contracting a nosocomial infection.

3. Method according to Claim 1 or 2, in which the biological sample was taken from a patient with an inflammatory systemic response, whether or not associated with an infection.

4. Method according to Claim 1 or 2, in which the biological sample is a blood sample.

5. Method according to Claim 1 or 2, in which the biological material is a nucleic acid or a protein.

6. Method according to any one of Claims 1 to 5, in which the specific reagent of the expression product of the S100A9 gene comprises at least one amplification primer specific to the S100A9 target gene.

7. Method according to any one of Claims 2 to 5, in which the specific reagent of the expression product of the S100A8 gene comprises at least one amplification primer specific to the S100A8 target gene.

8. Method according to any one of Claims 1 to 5, in which the specific reagent of the expression product of the S100A9 gene comprises at least one hybridization probe specific to the S100A9 target gene.

9. Method according to any one of Claims 2 to 5, in which the specific reagent of the expression product of the S100A8 gene comprises at least one hybridization probe specific to the S100A8 target gene.

10. Method according to any one of Claims 1 to 5, in which the specific reagent of the expression product of the S100A9 gene comprises at least one antibody specific to the molecule S100A9.

11. Method according to any one of Claims 2 to 5, in which the specific reagent of the expression product of the S100A8 gene comprises at least one antibody specific to the molecule S100A8.
